(19) Europäisches Patentamt
European Patent Office
Office européen des brevets

(11) Publication number: 0 242 939 B1

(12) EUROPEAN PATENT SPECIFICATION

(45) Date of publication of patent specification: 08.05.91 Bulletin 91/19

(51) Int. Cl.$^5$: C07D 209/14, A61K 31/40

(21) Application number: 87300741.3

(22) Date of filing: 28.01.87

# (54) Indole derivatives.

(30) Priority: 28.01.86 GB 8601959

(43) Date of publication of application: 28.10.87 Bulletin 87/44

(45) Publication of the grant of the patent: 08.05.91 Bulletin 91/19

(84) Designated Contracting States: AT BE CH DE ES FR GB IT LI NL SE

(56) References cited:
EP-A- 0 145 459
EP-A- 0 147 107
GB-A- 2 124 210
GB-A- 2 150 932
GB-A- 2 162 522
E.Mutschler, Arzneimittelwirkungen, 1981, p. 28-30

(73) Proprietor: GLAXO GROUP LIMITED
Clarges House 6-12 Clarges Street
London W1Y 8DH (GB)

(72) Inventor: Bays, David Edmund
9 Windmill Field
Ware Hertfordshire (GB)
Inventor: Oxford, Alexander William
60 Green Drift
Royston, Hertfordshire (GB)

(74) Representative: Kyle, Diana et al
ELKINGTON AND FIFE Beacon House 113 Kingsway
London WC2B 6PP (GB)

EP 0 242 939 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to indole derivatives of use in the treatment of migraine, to processes for their preparation, to pharmaceutical compositions containing them and to their medical use. The pain of migraine is associated with excessive dilatation of the cranial vasculature and known treatments for migraine include the administration of compounds having vasoconstrictor properties such as ergotamine. However, ergotamine is a non-selective vasoconstrictor which constricts blood vessels throughout the body and has undesirable and potentially dangerous side effects. Migraine may also be treated by administering an analgesic, usually in combination with an antiemetic, but such treatments are of limited value.

There is thus a need for a safe and effective drug for the treatment of migraine, which can be used either prophylactically or to alleviate an established headache, and a compound having a selective vasoconstrictor activity would fulfil such a role.

Furthermore, in conditions such as migraine, where the drug will usually be administered by the patient, it is highly desirable that the drug can be taken orally. It should therefore possess good bioavailability and be effectively absorbed from the gastro-intestinal tract so that prompt relief of symptoms can occur.

A wide variety of indole derivatives have been described as being of use in the treatment of migraine. In our published UK Patent Application No. 2124210A we describe indoles of the general formula

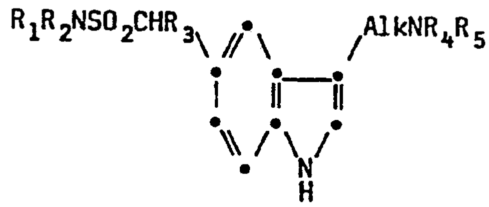

wherein $R_1$ represents a hydrogen atom or a $C_{1-6}$ alkyl or $C_{3-6}$ alkenyl group ; $R_2$ represents a hydrogen atom or a $C_{1-3}$ alkyl, $C_{3-6}$ alkenyl, aryl, ar($C_{1-4}$)alkyl or $C_{5-7}$ cycloalkyl group ; $R_3$ represents a hydrogen atom or a $C_{1-3}$ alkyl group ; $R_4$ and $R_5$, which may be the same or different, each represents a hydrogen atom or a $C_{1-3}$ alkyl or propenyl group or $R_4$ and $R_5$ together form an aralkylidene group ; and Alk represents an alkylene chain containing two or three carbon atoms which may be unsubstituted or substituted by not more than two $C_{1-3}$ alkyl groups, and physiologically acceptable salts and solvates thereof.

As indicated in UK Patent Application No. 2124210A, compounds of the above formula selectively constrict the carotid arterial bed of the anaesthetised dog and are thus potentially useful for the treatment of migraine.

Preferred compounds described in published UK Patent Application 2124210A include 3-(2-(methylamino)ethyl)-N-methyl-1H-indole-5-methanesulphonamide ; 3-(2-aminoethyl)-N,N-dimethyl-1H-indole-5-methanesulphonamide ; and 3-(2-aminoethyl)-N-(2-propenyl)-1H-indole-5-methanesulphonamide ; and their physiologically acceptable salts and solvates, and a particularly preferred compound described in that specification is 3-(2-aminoethyl)-N-methyl-1H-indole-5-methanesulphonamide, and its physiologically acceptable salts and solvates.

In our published UK Patent Application No. 2162522A we describe a particular compound which falls within the scope of the group of compounds claimed in published UK Patent Application No. 2124210A, but which is not specifically disclosed therein, namely 3-[2-(dimethylamino)ethyl]-N-methyl-1H-indole-5-methanesulphonamide, and its physiologically acceptable salts and solvates. This compound possesses a combination of highly advantageous properties for the treatment of migraine and in this respect has advantages over compounds specifically disclosed in published UK Patent Application 2124210A. Tests in anaesthetised dogs have shown that it potently and selectively constricts the carotid arterial bed following intravenous administration, and also that it is effectively and consistently well absorbed from the gastro-intestinal tract following intraduodenal administration. It's potent and selective vasoconstrictor action has also been demonstrated in vitro.

We have now found that certain 1-acyl derivatives of the above indoles exhibit highly potent and selective vasoconstrictor activity following administration to the gastro-intestinal tract.

Thus, the present invention provides an indole of general formula (I) :

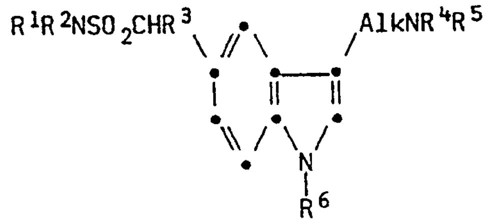

(I)

wherein $R^1$ represents a hydrogen atom or a $C_{1-6}$ alkyl or $C_{3-6}$ alkenyl group ;

$R^2$ represents a hydrogen atom, a $C_{1-3}$ alkyl or $C_{3-6}$ alkenyl group, a phenyl or phen($C_{1-4}$)alkylene group or a $C_{5-7}$ cycloalkyl group ;

$R^3$ represents a hydrogen atom or a $C_{1-3}$ alkyl group ;

$R^4$ and $R^5$, which may be the same or different, each represents a hydrogen atom or a $C_{1-3}$ alkyl or 2-propenyl group, or $R^4$ and $R^5$ together form a benzylidene group ;

$R^6$ represents a group $-CO_2R^7$, $COR^7$, $-COCO_2R^7$, or $-CONHR^7$, where $R^7$ represents a hydrogen atom, a $C_{1-4}$ alkyl group, a $C_{3-7}$ cycloalkyl group, a $C_{2-4}$ alkenyl group, or a phenyl or phen($C_{1-4}$) alkylene group wherein the phenyl group may be unsubstituted, or substituted by a halogen atom, a $C_{1-4}$ alkyl group, a hydroxy group or a $C_{1-4}$ alkoxy group (with the provisos that (a) $R^7$ does not represent a hydrogen atom or a benzyl group when $R^6$ is the group $-CO_2R^7$ and (b) $R^7$ does not represent an alkenyl group when $R^6$ is the group $-CONHR^7$) ; and Alk represents an alkylene chain containing two or three carbon atoms which may be unsubstituted or substituted by not more than two $C_{1-3}$ alkyl groups, and physiologically acceptable salts and solvates (e.g. hydrates) thereof.

Referring to the general formula (I), an alkyl group may be a straight or branched chain alkyl group preferably containing from 1 to 3 carbon atoms such as a methyl, ethyl, propyl or isopropyl group. An alkenyl group preferably contains 3 or 4 carbon atoms and may be, for example, a propenyl, 2-propenyl or butenyl group. It will be appreciated that when $R^1$ or $R^2$ represents a $C_{3-6}$ alkenyl group, the double bond will not be adjacent to the nitrogen atom.

A cycloalkyl group in compounds of formula (I) preferably contains from 5 to 7 carbon atoms and may be, for example, a cyclopentyl, cyclohexyl or cycloheptyl group.

The alkyl moiety in a phen($C_{1-4}$)alkylene group preferably contains 1 or 2 carbon atoms.

For the substituent $R^7$, when this is a $C_{1-4}$ alkyl group, it may be for example a methyl, ethyl, propyl, isopropyl or butyl group. When $R^7$ represents a $C_{3-7}$ cycloalkyl group this may be, for example a cyclopropyl, cyclopentyl or cyclohexyl group.

When $R^7$ represents a $C_{2-4}$ alkenyl group this may be for example a propenyl, butenyl or isobutenyl group. When $R^7$ represents a phen($C_{1-4}$)alkylene group, the alkyl moiety of the group may be a straight chain or branched chain alkyl moiety and is preferably a methyl or ethyl moiety. The alkyl moiety in a $C_{1-4}$ alkoxy group may be a straight or branched chain alkyl moiety and is preferably a methyl or ethyl moiety.

A preferred class of compounds represented by formula (I) is that in which $R^1$ represents a hydrogen atom or a $C_{1-6}$ alkyl group and $R^2$ represents a hydrogen atom or a $C_{1-3}$ alkyl, $C_{3-6}$ alkenyl or ar($C_{1-4}$)alkyl group.

Another preferred class of compounds of formula (I) is that in which $R^3$ represents a hydrogen atom.

A further preferred class of compounds of formula (I) is that in which $R^4$ and $R^5$, which may be the same or different, each represents a hydrogen atom or a $C_{1-3}$ alkyl group, for example a methyl or ethyl group. It is preferred that the total number of carbon atoms in $R^4$ and $R^5$ does not exceed two.

Another preferred class of compounds of formula (I) is that in which $R^6$ represents a group $-CO_2R^7$ or $-COR^7$.

A further preferred class of compounds represented by formula (I) is that in which $R^7$ represents a $C_{1-3}$ alkyl group, for example a methyl or ethyl group, or a phenyl group.

A still further preferred class of compounds falling within the scope of formula (I) is that wherein $R^1$ represents a methyl group, $R^2$ and $R^3$ both represent a hydrogen atom, $R^4$ and $R^5$ both represent a methyl group and $R^7$ represents a methyl, ethyl or phenyl group. Particularly important compounds within this group are those in which $R^6$ represents the group $-COR^7$ or $-CO_2R^7$, and physiologically acceptable salts and solvates (for example, hydrates) thereof.

Preferred compounds according to the invention include :

Methyl 3-[2-(dimethylamino)ethyl]-5-[[(methylamino)sulphonyl]methyl]-1H-indole-1-carboxylate ;

1-Acetyl-3-[2-(dimethylamino)ethyl]-N-methyl-1H-indole-5-methanesulphonamide ;

and physiologically acceptable salts and solvates (for example, hydrates) thereof.

Suitable physiologically acceptable salts of the indoles of general formula (I) include acid addition salts formed with organic or inorganic acids for example hydrochlorides, hydrobromides, sulphates, fumarates, maleates and succinates. Other salts may be useful in the preparation of the compounds of general formula (I) e.g. creatinine sulphate adducts and oxalates.

We have found that compounds of the invention potently and selectively constrict the carotid arterial bed of the anaesthetised dog following intraduodenal administration, whilst having negligible effect on blood pressure. However, the compounds produce no change in carotid vascular resistance following intravenous administration and exhibit no significant vasoconstrictor activity in standard in vitro tests. It is believed that following administration to the gastro-intestinal tract compounds of the invention are converted into the corresponding 1-unsubstituted indoles, i.e. they are prodrugs for the compounds disclosed in published UK Patent Application No. 2124210A and published UK Patent Application No. 2162522A.

Compounds of the invention are therefore useful in treating pain resulting from dilatation of the cranial vasculature, in particular migraine and cluster headache.

Compounds of the invention are suitable for oral, rectal or intranasal administration.

Accordingly the invention provides a pharmaceutical composition adapted for use in medicine which comprises at least one compound of formula (I) or a physiologically acceptable salt or solvate (e.g. hydrate) thereof and which is formulated for oral or rectal administration. Such compositions may be formulated in conventional manner using one or more pharmaceutically acceptable carriers or excipients.

The pharmaceutical compositions for oral administration may take the form of, for example, tablets or capsules prepared by conventional means with pharmaceutically acceptable excipients such as binding agents (e.g. pregelatinised maize starch, polyvinylpyrrolidone or hydroxypropyl methylcellulose) ; fillers (e.g. lactose, sucrose, mannitol, maize starch, microcrystalline cellulose or calcium hydrogen phosphate) ; lubricants (e.g. stearic acid, polyethylene glycol, magnesium stearate, talc or silica) ; disintegrants (e.g. potato starch, sodium starch glycollate or croscarmellose sodium) ; or wetting agents (e.g. sodium lauryl sulphate). The tablets may be coated by methods well known in the art. Liquid preparations for oral administration may take the form of, for example, aqueous or oily solutions, syrups, elixirs, emulsions or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives, glucose/sugar syrup, gelatin, aluminium stearate gel, or hydrogenated edible fats) ; emulsifying agents (e.g. lecithin, acacia or sorbitan mono-oleate) ; non-aqueous vehicles (e.g. almond oil, oily esters, ethyl alcohol or fractionated vegetable oils) ; and preservatives (eg. methyl or propyl p-hydroxybenzoates or sorbic acid). The liquid preparations may also contain conventional buffers, flavouring, colouring and sweetening agents as appropriate.

For rectal administration the compounds of the invention may be formulated as suppositories or retention enemas e.g. containing conventional suppository bases such as cocoa butter or other glycerides.

A proposed dose of the compounds of the invention for administration to man (about 70 kg bodyweight) for the treatment of migraine is 1 mg to 1000 mg, for example 3 mg to 300 mg of the active ingredient per unit dose, which could be administered for example 1 to 4 times per day. It will be appreciated that it may be necessary to make routine variations to the dosage depending on the age and weight of the patient, as well as the severity of the condition to be treated.

According to another aspect of the invention, compounds of general formula (I) and their physiologically acceptable salts and solvates (e.g. hydrates) may be prepared by the general methods outlined hereinafter. In the following processes $R^1$, $R^2$, $R^3$, $R^4$, $R^5$, $R^6$ and Alk are as defined for the general formula (I) unless otherwise specified.

According to one general process (A), compounds of general formula (I) may be prepared by acylating a compound of general formula (II) :

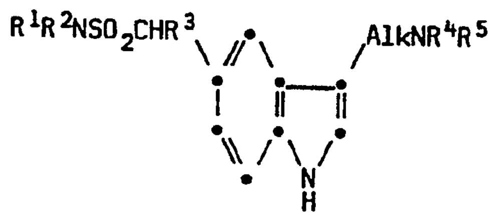

(II)

or a protected derivative thereof.

Acylating agents corresponding to the group $R^6$ which may be used in this general process include acid halides (e.g. acid chlorides such as acetyl chloride) ; alkyl haloformates (e.g. methyl or ethyl chloroformate) ;

mixed or symmetrical anhydrides (e.g. acetic anhydride or benzoic anhydride) ; carbonates (e.g. ethyl carbonate) ; and isocyanates (e.g. methyl isocyanate).

The reaction is conveniently effected in the presence of a base, such as an alkali metal hydride, e.g. sodium or potassium hydride ; an alkali metal carbonate e.g.. sodium or potassium carbonate ; an alkali metal alkoxide e.g. potassium t-butoxide ; butyllithium ; or an organic tertiary amine, e.g. triethylamine, or pyridine.

Suitable solvents which may be employed in the acylation process include amides e.g. dimethylformamide, or dimethylacetamide ; ethers, e.g. tetrahydrofuran or dioxan ; halogenated hydrocarbons e.g. methylene chloride ; nitriles e.g. acetonitrile and esters e.g. ethyl acetate. The reaction may conveniently be effected at a temperature in the range −10 to +150°C.

Alternatively the acylation may be effected in a two-phase reaction medium, in the presence of a phase transfer catalyst, such as tetrabutylammonium hydrogen sulphate or tetraethylammonium bromide. Thus for example the acylating agent may be reacted with a compound of formula (II) in an inert organic solvent, (e.g. a halogenated hydrocarbon such as methylene chloride), and an aqueous solution of a base (e.g. 50% sodium hydroxide) containing a phase transfer catalyst.

Compounds of general formula (II) may be prepared for example by the methods described in published UK Patent Application 2124210A.

According to a further general process (B) a compound of formula (I) according to the invention, or a salt or protected derivative thereof may be converted into another compound of the invention using conventional procedures.

For example, a compound of general formula (I) wherein one or more of $R^1$, $R^2$, $R^4$ and $R^5$ are alkyl groups may be prepared from the corresponding compounds of formula (I) wherein one or more of $R^1$, $R^2$, $R^4$ and $R^5$ represent hydrogen atoms, by reaction with a suitable alkylating agent such as a compound of formula $R^cL$ where $R^c$ represents the desired $R^1$, $R^2$, $R^4$ or $R^5$ group and L represents a leaving group such as a halogen atom or a tosylate group, or a sulphate $(R^c)_2SO_4$. Thus, the alkylating agent may be for example an alkyl halide (e.g. methyl or ethyl iodide), alkyl tosylate (e.g. methyl tosylate) or dialkylsulphate (e.g. dimethylsulphate). The alkylation reaction is conveniently carried out in an inert organic solvnt such as an amide (e.g. dimethylformamide), an ether (e.g. tetrahydrofuran) or an aromatic hydrocarbon (e.g. toluene) preferably in the presence of a base. Suitable bases include, for example, alkali metal hydrides, such as sodium or potassium hydride, alkali metal amides, such as sodium amide, alkali metal carbonates, such as sodium carbonate ; alkali metal alkoxides such as sodium or potassium methoxide, ethoxide or t-butoxide ; and tetrabutylammonium fluoride. When an alkyl halide is employed as the alkylating agent the reaction may also be carried out in the presence of an acid scavenger such as propylene or ethylene oxide. The reaction may be conveniently effected at a temperature of −20°C to +100°C.

Compounds of formula (I) wherein $R^1$ represents a $C_{3-6}$ alkenyl group, $R^2$ represents a $C_{3-6}$ alkenyl, ar($C_{1-4}$)alkyl or $C_{5-7}$ cycloalkyl group and/or one or both of $R^4$ and $R^5$ represents propenyl may be prepared similarly, using an appropriate compound of formula $R^cL$ or $(R^c)_2SO_4$.

According to another general process (C), a compound of general formula (I) according to the invention, or a salt thereof may be prepared by subjecting a protected derivative of general formula (I) or a salt thereof to reaction to remove the protecting group or groups.

Thus, at an earlier stage in the procedure for the preparation of a compound of general formula (I) or a salt thereof it may have been necessary or desirable to protect one or more sensitive groups in the molecule to avoid undesirable side reactions. For example it may be necessary to protect the group $NR^4R^5$, wherein $R^4$ and/or $R^5$ represents hydrogen, with a group easily removable at the end of the reaction sequence.

Such protection may be effected in conventional manner, for example as described in "Protective Groups in Organic Chemistry" Ed. J.F.W. Mc Omie (Plenum Press 1973) or "Protective Groups in Organic Synthesis" by Theodora W. Greene (John Wiley and Sons 1981). It will be appreciated that the protecting group should be one which can be removed under conditions which do not cleave the acyl group $R^6$. Thus, for example it may be an aralkyl group such as benzyl which may be cleaved by hydrogenolysis in the presence of a catalyst (e.g. palladian on charcoal).

As will be appreciated, in either of the general processes (A) or (B) described previously it may be necessary or desirable to protect any sensitive groups in the molecule as just described. Thus, a reaction step involving deprotection of a protected derivative of general formula (I) or a salt thereof may be carried out subsequent to either of the previously described proceses (A) or (B).

Thus, according to a further aspect of the invention, the following reactions in any appropriate sequence may if necessary and/or desired be carried out subsequent to either of the processes (A) or (B) :

(i) removal of any protecting groups ; and

(ii) conversion of a compound of general formula (I) or a salt thereof into a physiologically acceptable salt or solvate (e.g. hydrate) thereof.

Where it is desired to isolate a compound of the invention as a physiologically acceptable salt, for example as an acid addition salt, this may be achieved by treating the free base of general formula (I), with an appropriate acid (e.g. succinic or hydrochloric acid) preferably with an equivalent amount in a suitable solvent (e.g. aqueous ethanol).

As well as being employed as the last main step in the preparative sequence, the general methods indicated above for the preparation of the compounds of the invention may also be used for the introduction of the desired groups at an intermediate stage in the preparation of the required compound. Thus, for example, the required group at the 5-position may be introduced either before or after cyclisation to form the indole nucleus. It should therefore be appreciated that in such multi-stage processes, the sequence of reactions should be chosen in order that the reaction conditions do not affect groups present in the molecule which are desired in the final product.

The following Examples illustrate the invention. All temperatures are in °C.

Example 1

Ethyl 3-[2-(dimethylamino)ethyl]-5-[[(methylamino)sulphonyl]methyl]-1H-indole-1-carboxylate oxalate

A stirred solution of 3-[2-(dimethylamino)ethyl]-N-methyl-1H-indole-5-methanesulphonamide (0.5 g) in dimethylformamide (10 ml) was treated with sodium hydride (0.16 g, 80% dispersion in oil) and stirred for 0.5h at room temperature. The solution was cooled to 0° and ethyl chloroformate (0.183 g, 0.16 ml) was added dropwise. The mixture was stirred for 1h at room temperature, quenched with sodium bicarbonate (20 ml) and extracted with ethyl acetate (3 × 30 ml). The organic extracts were washed with brine (2 × 30 ml), dried ($MgSO_4$) and concentrated in vacuo to give an oil (0.45 g) which was purified by short path chromatography (Merck silica 7747, 20 g) eluting with dichloromethane : ethanol : ammonia (100 : 8 : 1) to give a solid (0.24 g) which was triturated with diethyl ether (20 ml) to give a powder (0.143 g). The powder was dissolved in hot ethanol (5 ml) and heated with a hot solution of oxalic acid (38 mg) in ethanol (1 ml). The solution was allowed to cool and the crystals that formed were collected and dried at 60° in vacuo to give the title compound (0.130 g) m.p. 197-198°C

T.l.c. Silica (dichloromethane : ethanol : ammonia = 50 : 8 : 1) Rf 0.8 detection u.v., $KMnO_4$

| | | |
|---|---|---|
| Analysis | Found : | C,49.8;H,6.4;N,8.8. |
| $C_{17}H_{25}N_3O_4S.C_2H_2O_4.0.14\ H_2O$ requires: | | C,49.6;H,6.0;N,9.1%. |

$H_2O$ analysis : 0.55% water content ≡ 0.14mol $H_2O$.

Example 2

Methyl 3-[2-(dimethylamino)ethyl]-5-[[(methylamino)sulphonyl]methyl]-1H-indole-1-carboxylate oxalate

A solution of 3-[2-(dimethylamino)ethyl]-N-methyl-1H-indole-5-methane sulphonamide (1.0 g), in dimethylformamide (50 ml) was added to sodium hydride (0.2 g, 80% dispersion in oil) under a nitrogen atmosphere to give a suspension which was stirred for 2h at room temperature, then cooled to 5°. Methyl chloroformate (0.32 g, 0.26 ml), in tetrahydrofuran (5 ml) was added dropwise with cooling over 10 min. The reaction mixture was stirred for a further 1h at 5° and then poured into a mixture of chloroform (50 ml) and saturated ammonium chloride (50 ml).The chloroform layer was concentrated in vacuo, dissolved in ethyl acetate (50 ml) and backwashed with saturated brine (250 ml). The organic layer was evaporated in vacuo to give a solid which was purified by chromatography (activated alumina 90, Merck 45 g), eluting with dichloromethane : methanol (98 : 2). The appropriate fractions were combined and evaporated in vacuo to give the 1-acetyl derivative as a solid. The solid (0.445 g) was dissolved in hot absolute ethanol and treated with oxalic acid (113 mg) in methanol. The crystals (0.43 g) that formed were recrystallised from methanol (20 ml) and dried in vacuo to give the title compound (0.3 g) as a powder m.p. 185-186°.

T.l.c. Silica dichloromethane : ethanol : ammonia 50 : 8 : 1 Rf 0.5 detection u.v. IPA, $KMnO_4$.

| | |
|---|---|
| Analysis Found : | C,48.6; H,5.7; N,9.3. |
| $C_{16}H_{23}N_3O_4S.C_2H_2O_4$ requires: | C,48.4; H,5.7; N,9.4%. |

Example 3

1-Acetyl-3-[2-(dimethylamino)ethyl]-N-methyl-1H-indole-5-methanesulphonamide hemioxalate

A solution of 3-[2-(dimethylamino)ethyl]-N-methyl-1H-indole-5-methane sulphonamide (1.0 g) in dimethylformamide (50 ml) was added to sodium hydride (0.2 g, 80% dispersion in oil) under a nitrogen atmosphere to give a suspension which was stirred for 2h, then cooled to 5°. Acetyl chloride (0.26 g) in tetrahydrofuran (5 ml) was added dropwise over 15 min, maintaining the temperature below 5°. The reaction mixture was stirred for a further 1h, and then poured into a mixture of chloroform (50 ml) and saturated ammonium chloride (50 ml). The chloroform layer was concentrated in vacuo, dissolved in ethyl acetate (50 ml) and backwashed with saturated brine (250 ml). The organic layer was evaporated in vacuo and the resulting solid was triturated with diethyl ether (250 ml) to give a powder (0.44 g) which was dissolved in ethanol (25 ml), treated with oxalic acid (119 mg) in methanol (25 ml) and dried in vacuo to give the title compound (0.15 g) as a solid m.p. 208-210°.
T.l.c. Silica dichloromethane :ethanol :ammonia 50 : 8 : 1 Rf 0.6

Analysis Found: C,52.4; H,6.3; N,10.4
$C_{16}H_{23}N_3O_3S.0.5C_2H_2O_4.0.3H_2O$ requires : C,52.7; H,6.3; N,10.8.
$H_2O$ Assay indicates 1.28% $H_2O$ = 0.3mol $H_2O$.

Example 4

1-Benzoyl-3-[2-(dimethylamino)ethyl]-N-methyl-1H-indole-5-methanesulphonamide oxalate

Benzoic acid anhydride (0.382 g) was dissolved in pyridine (5 mℓ), cooled to −5° and treated dropwise with a solution of 3-[2-(dimethylamino)ethyl]-N-methyl-1H-indole-5-methanesulphonamide (0.5 g) in pyridine under nitrogen. The solution was heated at reflux for 2h, treated with saturated ammonium chloride and extracted with ethyl acetate. The organic phase was separated and concentrated in vacuo to give an oil which was purified by chromatography (Merck Silica 9385) and eluted with a mixture of dichloromethane : ethanol : ammonia (150: 8 : 1). The appropriate fractions were combined and concentrated in vacuo to give a solid (0.1 g) which was dissolved in ethanol (20 mℓ) and treated with oxalic acid (31 mg, 1 equivalent) in methanol (2 mℓ). The crystals that formed were collected and dried at 70° in vacuo for 18h to give the title compound (0.075 g) as a solid m.p. 205-208°.
T.l.c. Silica dichloromethane :ethanol :ammonia 150 : 8 : 1 Rf 0.3 detection IPA, $KMnO_4$

Assay Found: C,56.2; H,5.6; N,8.2.
$C_{21}H_{25}N_3O_3S.C_2H_2O_4.0.2H_2O$ requires C,56.0; H,5.6; N,8.5%.
$H_2O$ assay contains 0.76 $H_2O$ w/w≡0.2mol $H_2O$

The following example illustrates a pharmaceutical formulation according to the invention containing 1-acetyl-3-[2-(dimethylamino)ethyl]-N-methyl-1H-indole-5-methanesulphonamide hemioxalate as the active ingredient. Other compounds of the invention may be formulated in a similar manner.

Tablets for Oral Administration

| | mg/tablet |
|---|---|
| Active Ingredient | 100 |
| Magnesium stearate BP | 1.0 |
| Anhydrous lactose | 99 |

The active ingredient is sieved and blended with the anhydrous lactose and magnesium stearate. The mix is then compressed into tablets using a Manesty F3 (Trade Mark) tablet machine fitted with 8.0 mm concave punches.

## Claims

### Claims for the Contracting States BE CH DE FR GB IT LI NL SE :

1. A compound of general formula (I) :

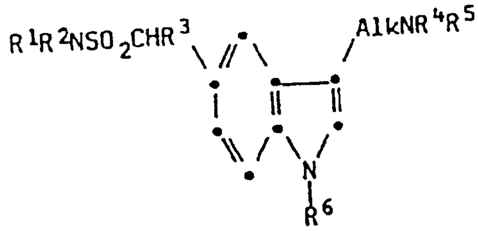

(I)

wherein $R^1$ represents a hydrogen atom or a $C_{1-6}$ alkyl or $C_{3-6}$ alkenyl group ;
$R^2$ represents a hydrogen atom, a $C_{1-3}$ alkyl or $C_{3-6}$ alkenyl group, a phenyl or phen($C_{1-4}$)alkylene group, or a $C_{5-7}$ cycloalkyl group ;
$R^3$ represents a hydrogen atom or a $C_{1-3}$ alkyl group ;
$R^4$ and $R^5$ which may be the same or different, each represents a hydrogen atom or a $C_{1-3}$ alkyl or 2-propenyl group, or $R^4$ and $R^5$ together form a benzylidene group ;
$R^6$ represents a group $-CO_2R^7$, $COR^7$, $-COCO_2R^7$, or $-CONHR^7$, where $R^7$ represents a hydrogen atom, a $C_{1-4}$ alkyl group, a $C_{3-7}$ cycloalkyl group, a $C_{2-4}$ alkenyl group, or a phenyl or phen($C_{1-4}$)alkylene group (with the provisos that (a) $R^7$ does not represent a hydrogen atom or a benzyl group when $R^6$ is the group $-CO_2R^7$ and (b) $R^7$ does not represent an alkenyl group when $R^6$ is the group $-CONHR^7$) ; and Alk represents an alkylene chain containing two or three carbon atoms which may be unsubstituted or substituted by not more than two $C_{1-3}$ alkyl groups, and physiologically acceptable salts and solvates thereof.

2. A compound according to claim 1, wherein, in the general formula (I) $R^1$ represents a hydrogen atom or a $C_{1-6}$ alkyl group and $R^2$ represents a hydrogen atom or a $C_{1-3}$ alkyl, $C_{3-6}$ alkenyl or phen($C_{1-4}$) alkyl group.

3. A compound according to claim 1 or 2, wherein, in the general formula (I), $R^3$ represents a hydrogen atom.

4. A compound according to any of claims 1 to 3, wherein, in the general formula (I), $R^4$ and $R^5$, which may be the same or different, each represents a hydrogen atom or a $C_{1-3}$ alkyl group.

5. A compound according to any of claims 1 to 4, wherein, in the general formula (I), $R^6$ represents a group $-CO_2R^7$ or $-COR^7$ (where $R^7$ is as defined in claim 1).

6. A compound according to any of claims 1 to 5, wherein, in the general formula (I), $R^7$ represents a $C_{1-3}$ alkyl group or a phenyl group.

7. A compound according to claim 1, wherein, in the general formula (I), $R^1$ represents a methyl group, $R^2$ and $R^3$ both represent a hydrogen atom, $R^4$ and $R^5$ both represent a methyl group and $R^7$ represents a methyl, ethyl or phenyl group.

8. A compound according to claim 1 selected from methyl 3-[2-(dimethylamino)ethyl]-5-[[(methylamino)sulphonyl]methyl]-1H-indole-1-carboxylate ;
1-acetyl-3-[2-(dimethylamino)ethyl]-N-methyl-1H-indole-5-methane-sulphonamide.
and physiologically acceptable salts and solvates thereof.

9. A pharmaceutical composition suitable for oral, rectal or intranasal administration comprising at least one compound of general formula (I) as defined in claim 1, or a physiologically acceptable salt or solvate thereof together with a physiologically acceptable carrier or excipient therefor.

10. A process for the preparation of a compound of general formula (I) as defined in claim 1 which comprises :

(A) acylating a compound of general formula (II) :

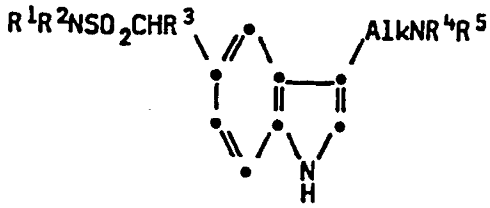

(II)

(wherein $R^1$, $R^2$, $R^3$, $R^4$ and $R^5$ are as defined in claim 1)
or a protected derivative thereof ; or
(B) subjecting a compound of general formula (I) or a salt or protected derivative thereof to an inter-conversion reaction to form another compound of general formula (I) or a physiologically acceptable salt or protected derivative thereof ; or
(C) subjecting a protected derivative of a compound of general formula (I) or a salt thereof to reaction to remove the protecting group or groups to prepare a compound of general formula (I) or a physiologically acceptable salt thereof ; and, if necessary or desired, subjecting a compound prepared by step (A) or step (B) to one or two further reactions comprising
(i) removing any protecting groups ; and
(ii) converting a compound of general formula (I) or a salt thereof into a physiologically acceptable salt or solvate thereof.

**Claims for the Contracting States AT ES :**

1. A process for the preparation of a compound of general formula (I) :

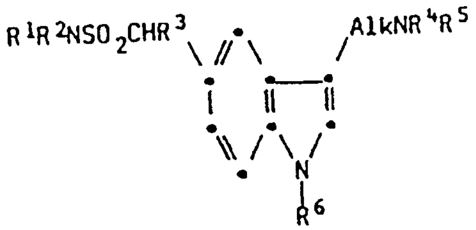

(I)

wherein $R^1$ represents a hydrogen atom or a $C_{1-6}$ alkyl or $C_{3-6}$ alkenyl group ;
$R^2$ represents a hydrogen atom, a $C_{1-3}$ alkyl or $C_{3-6}$ alkenyl group, a phenyl or phen($C_{1-4}$)alkylene group, or a $C_{5-7}$ cycloalkyl group ;
$R^3$ represents a hydrogen atom or a $C_{1-3}$ alkyl group ;
$R^4$ and $R^5$ which may be the same or different, each represents a hydrogen atom or a $C_{1-3}$ alkyl or 2-propenyl group, or $R^4$ and $R^5$ together form a benzylidene group ;
$R^6$ represents a group $-CO_2R^7$, $COR^7$, $-COCO_2R^7$, or $-CONHR^7$, where $R^7$ represents a hydrogen atom, a $C_{1-4}$ alkyl group, a $C_{3-7}$ cycloalkyl group, a $C_{2-4}$ alkenyl group, or a phenyl or phen($C_{1-4}$)alkylene group (with the provisos that (a) $R^7$ does not represent a hydrogen atom or a benzyl group when $R^6$ is the group $-CO_2R^7$ and (b) $R^7$ does not represent an alkenyl group when $R^6$ is the group $-CONHR^7$) ; and
Alk represents an alkylene chain containing two or three carbon atoms which may be unsubstituted or substituted by not more than two $C_{1-3}$ alkyl groups,
or a physiologically acceptable salt or solvate thereof which comprises :
(A) acylating a compound of general formula (II) :

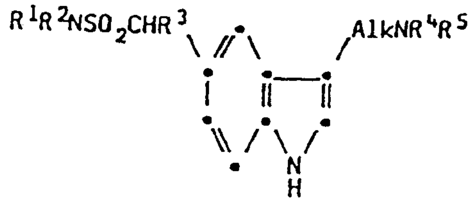

(II)

(wherein $R^1$, $R^2$, $R^3$, $R^4$ and $R^5$ are as defined for formula (I)) or a protected derivative thereof ; or

(B) subjecting a compound of general formula (I) or a salt or protected derivative thereof to an inter-conversion reaction to form another compound of general formula (I) or a physiologically acceptable salt or protected derivative thereof ; or

(C) subjecting a protected derivative of a compound of general formula (I) or a salt thereof to reaction to remove the protecting group or groups to prepare a compound of general formula (I) or a physiologically acceptable salt thereof ; and, if necessary or desired, subjecting a compound prepared by step (A) or step (B) to one or two further reactions comprising

(i) removing any protecting groups ; and

(ii) converting a compound of general formula (I) or a salt thereof into a physiologically acceptable salt or solvate thereof.

2. A process according to claim 1, wherein, in the general formula (I) $R^1$ represents a hydrogen atom or a $C_{1-6}$ alkyl group and $R^2$ represents a hydrogen atom or a $C_{1-3}$ alkyl, $C_{3-6}$ alkenyl or phen($C_{1-4}$) alkyl group.

3. A process according to claim 1 or 2, wherein, in the general formula (I), $R^3$ represents a hydrogen atom.

4. A process according to any of claims 1 to 3, wherein, in the general formula (I), $R^4$ and $R^5$, which may be the same or different, each represents a hydrogen atom or a $C_{1-3}$ alkyl group.

5. A process according to any of claims 1 to 4, wherein, in the general formula (I), $R^6$ represents a group $-CO_2R^7$ or $-COR^7$ (where $R^7$ is as defined in claim 1).

6. A process according to any of claims 1 to 5, wherein, in the general formula (I), $R^7$ represents a $C_{1-3}$ alkyl group or a phenyl group.

7. A process according to claim 1, wherein, in the general formula (I), $R^1$ represents a methyl group, $R^2$ and $R^3$ both represent a hydrogen atom, $R^4$ and $R^5$ both represent a methyl group and $R^7$ represents a methyl, ethyl or phenyl group.

8. A process according to claim 1, wherein the product is selected from methyl 3-[2-(dimethylamino)ethyl]-5-[[(methylamino)sulphonyl]methyl]-1H-indole-1-carboxylate ;
1-acetyl-3-[2-(dimethylamino)ethyl]-N-methyl-1H-indole-5-methane-sulphonamide ;
and physiologically acceptable salts and solvates thereof.

9. A process according to any of claims 1 to 8, wherein the acylation step (A) is effected in the presence of a base at a temperature of from −10 to +150°C in the presence of a solvent or in a two-phase reaction medium in the presence of a phase transfer catalyst.

10. A process according to any of claims 1 to 8, wherein in step (B) a compound of general formula (I) wherein one or more of $R^1$, $R^2$, $R^4$ and $R^5$ are alkyl groups is prepared from a corresponding compound of formula (I) wherein one or more of $R^1$, $R^2$, $R^4$ and $R^5$ represent hydrogen atoms by reaction with an alkylating agent.

## Ansprüche

**Patentansprüche für die Vertragssstaaten BE CH DE FR GB IT LI NL SE :**

1. Verbindung der allgemeinen Formel (I)

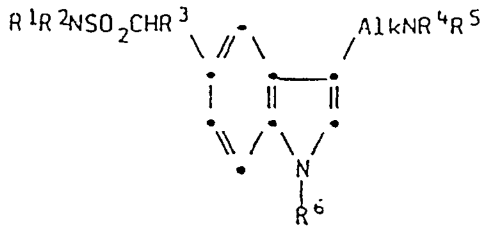

(I)

worin $R^1$ für ein Wasserstoffatom oder eine $C_{1-6}$-Alkyl- oder $C_{3-6}$-Alkenylgruppe steht ;
$R^2$ für ein Wasserstoffatom, eine $C_{1-3}$-Alkyl- oder $C_{3-6}$-Alkenylgruppe, eine Phenyl- oder Phen($C_{1-4}$)alkylengruppe oder eine $C_{5-7}$-Cycloalkylgruppe steht ;
$R^3$ für ein Wasserstoffatom oder eine $C_{1-3}$-Alkylgruppe steht ;
$R^4$ und $R^5$, die gleich oder verschieden sein können, jeweils für ein Wasserstoffatom oder für eine $C_{1-3}$-Alkyl oder 2-Propenylgruppe stehen oder $R^4$ und $R^5$ zusammen eine Benzylidengruppe bilden ;
$R^6$ für eine Gruppe $-CO_2R^7$, $COR^7$, $-COCO_2R^7$ oder $-CONHR^7$ steht, worin $R^7$ für ein Wasserstoffatom, eine $C_{1-4}$-Alkylgruppe, eine $C_{3-7}$-Cycloalkylgruppe, eine $C_{2-4}$-Alkenylgruppe oder eine Phenyl- oder Phen($C_{1-4}$)alkylengruppe steht (mit den Maßgaben daß (a) $R^7$ nicht für ein Wasserstoffatom oder eine Benzylgruppe steht, wenn $R^6$ die Gruppe $-CO_2R^7$ bedeutet, und (b) $R^7$ nicht für eine Alkenylgruppe steht, wenn $R^6$ die Gruppe $-CONHR^7$ bedeutet) ; und Alk für eine Alkylenkette aus zwei oder drei Kohlenstoffatomen steht, die unsubstituiert oder durch nicht mehr als zwei $C_{1-3}$-Alkylgruppen substituiert sein kann,
und physiologisch annehmbare Salze und Solvate davon.

2. Verbindung nach Anspruch 1, dadurch **gekennzeichnet,** daß in der allgemeinen Formel (I) $R^1$ für ein Wasserstoffatom oder eine $C_{1-6}$-Alkylgruppe steht und $R^2$ für ein Wasserstoffatom oder eine $C_{1-3}$-Alkyl-, $C_{3-6}$-Alkenyl- oder Phen($C_{1-4}$)alkylgruppe steht.

3. Verbindung nach Anspruch 1 oder 2, dadurch **gekennzeichnet,** daß in der allgemeinen Formel (I) $R^3$ für ein Wasserstoffatom steht.

4. Verbindung nach einem der Ansprüche 1 bis 3, dadurch **gekennzeichnet,** daß in der allgemeinen Formel (I) $R^4$ und $R^5$, die gleich oder verschieden sein können, jeweils für ein Wasserstoffatom oder eine $C_{1-3}$-Alkylgruppe stehen.

5. Verbindung nach einem der Ansprüche 1 bis 4, dadurch **gekennzeichnet,** daß in der allgemeinen Formel (I) $R^6$ für eine Gruppe $-CO_2R^7$ oder $-COR^7$ (worin $R^7$ wie in Anspruch 1 definiert ist) steht.

6. Verbindung nach einem der Ansprüche 1 bis 5, dadurch **gekennzeichnet,** daß in der allgemeinen Formel (I) $R^7$ für eine $C_{1-3}$-Alkylgruppe oder eine Phenylgruppe steht.

7. Verbindung nach Anspruch 1, dadurch **gekennzeichnet,** daß in der allgemeinen Formel (I) $R^1$ für eine Methylgruppe steht, $R^2$ und $R^3$ beide für ein Wasserstoffatom stehen, $R^4$ und $R^5$ beide für eine Methylgruppe stehen und $R^7$ für eine Methyl-, Ethyl- oder Phenylgruppe steht.

8. Verbindung nach Anspruch 1, dadurch **gekennzeichnet,** daß sie aus
Methyl-3-[2-(dimethylamino)ethyl]-5-[[(methylamino)-sulfonyl]methyl]-1H-indol-1-carboxylat ;
1-Acetyl-3-[2-(dimethylamino)ethyl]-N-methyl-1H-indol-5-methan-sulfonamid
und physiologisch annehmbaren Salzen und Solvaten davon ausgewählt ist.

9. Pharmazeutisches Präparat geeignet für die orale, rektale oder intranasale Verabreichung, dadurch **gekennzeichnet,** daß es mindestens eine Verbindung der allgemeinen Formel (I), wie in Anspruch 1 definiert, oder ein physiologisch annehmbares Salz oder Solvat davon zusammen mit einem physiologisch annehmbaren Träger oder Exzipienten dafür umfaßt.

10. Verfahren zur Herstellung einer Verbindung der allgemeinen Formel (I) wie in Anspruch 1 definiert, dadurch **gekennzeichnet**, daß man
(A) eine Verbindung der allgemeinen Formel (II)

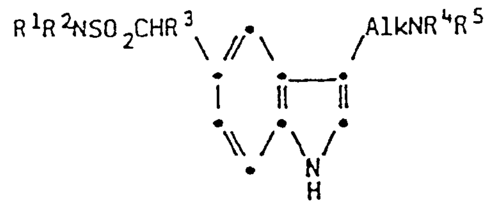

(II)

(worin $R^1$, $R^2$, $R^3$, $R^4$ und $R^5$ wie in Anspruch 1 definiert sind)
oder ein geschütztes Derivat davon acyliert ; oder
(B) eine Verbindung der allgemeinen Formel (I) oder ein Salz oder ein geschütztes Derivat davon einer Interconversionsreaktion unterwirft, um eine weitere Verbindung der allgemeinen Formel (I) oder ein physiologisch annehmbares Salz oder ein geschütztes Derivat davon zu bilden ; oder
(C) ein geschütztes Derivat einer Verbindung der allgemeinen Formel (I) oder ein Salz davon einer Reaktion zur Entfernung der Schutzgruppe oder der Schutzgruppen unterwirft, um eine Verbindung der allgemeinen Formel (I) oder ein physiologisch annehmbares Salz davon herzustellen ; und, sofern notwendig oder zweckmäßig, eine Verbindung, hergestellt gemäß Stufe (A) oder Stufe (B), einer oder zwei weiteren Reaktionen unterwirft, bei denen man
(i) alle Schutzgruppen entfernt ; und
(ii) eine Verbindung der allgemeinen Formel (I) oder ein Salz davon in ein physiologisch annehmbares Salz oder Solvat davon umwandelt.

**Patentansprüche für die Vertragssstaaten AT ES :**

1. Verfahren zur Herstellung einer Verbindung der allgemeinen Formel (I)

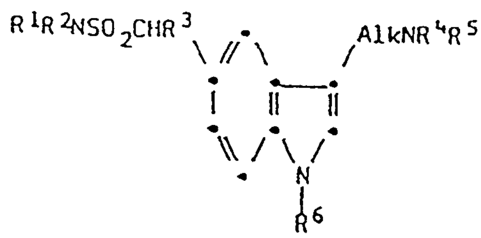

(I)

worin $R^1$ für ein Wasserstoffatom oder eine $C_{1-6}$-Alkyl- oder $C_{3-6}$-Alkenylgruppe steht ;
$R^2$ für ein Wasserstoffatom, eine $C_{1-3}$-Alkyl- oder $C_{3-6}$-Alkenylgruppe, eine Phenyl- oder Phen($C_{1-4}$)alkylengruppe oder eine $C_{5-7}$-Cycloalkylgruppe steht ;
$R^3$ für ein Wasserstoffatom oder eine $C_{1-3}$-Alkylgruppe steht ;
$R^4$ und $R^5$, die gleich oder verschieden sein können, jeweils für ein Wasserstoffatom oder für eine $C_{1-3}$-Alkyl oder 2-Propenylgruppe stehen oder $R^4$ und $R^5$ zusammen eine Benzylidengruppe bilden ;
$R^6$ für eine Gruppe $-CO_2R^7$ , $COR^7$, $-COCO_2R^7$ oder $-CONHR^7$ steht, worin $R^7$ für ein Wasserstoffatom, eine $C_{1-4}$-Alkylgruppe, eine $C_{3-7}$-Cycloalkylgruppe, eine $C_{2-4}$-Alkenylgruppe oder eine Phenyl- oder Phen($C_{1-4}$)alkylengruppe steht (mit der Maßgabe daß (a) $R^7$ nicht für ein Wasserstoffatom oder eine Benzylgruppe steht, wenn $R^6$ die Gruppe $-CO_2R^7$ bedeutet, und (b) $R^7$ nicht für eine Alkenylgruppe steht, wenn $R^6$ die Gruppe $-CONHR^7$ bedeutet) ; und Alk für eine Alkylenkette aus zwei oder drei Kohlenstoffatomen steht, die unsubstituiert oder durch nicht mehr als zwei $C_{1-3}$-Alkylgruppen substituiert sein kann,
oder physiologisch annehmbare Salze oder Solvate davon, dadurch **gekennzeichnet**, daß man
(A) eine Verbindung der allgemeinen Formel (II)

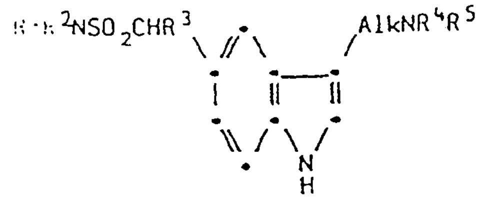

(II)

(worin $R^1$, $R^2$, $R^3$, $R^4$ und $R^5$ wie in Anspruch 1 definiert sind) oder ein geschütztes Derivat davon acyliert ; oder

(B) eine Verbindung der allgemeinen Formel (I) oder ein Salz oder ein geschütztes Derivat davon einer Interconversionsreaktion unterwirft, um eine weitere Verbindung der allgemeinen Formel (I) oder ein physiologisch annehmbares Salz oder ein geschütztes Derivat davon zu bilden ; oder

(C) ein geschütztes Derivat einer Verbindung der allgemeinen Formel (I) oder ein Salz davon einer Reaktion zur Entfernung der Schutzgruppe oder der Schutzgruppen unterwirft, um eine Verbindung der allgemeinen Formel (I) oder ein physiologisch annehmbares Salz davon herzustellen ; und, sofern notwendig oder zweckmäßig, eine Verbindung, hergestellt gemäß Stufe (A) oder Stufe (B), einer oder zwei weiteren Reaktionen unterwirft, bei denen man

(i) alle Schutzgruppen entfernt ; und

(ii) eine Verbindung der allgemeinen Formel (I) oder ein Salz davon in ein physiologisch annehmbares Salz oder Solvat davon umwandelt.

2. Verfahren nach Anspruch 1, dadurch **gekennzeichnet,** daß in der allgemeinen Formel (I) $R^1$ für ein Wasserstoffatom oder eine $C_{1-6}$-Alkylgruppe steht, $R^2$ für ein Wasserstoffatom oder eine $C_{1-3}$-Alkyl-, $C_{3-6}$-Alkenyl- oder Phen($C_{1-4}$)alkylgruppe steht.

3. Verfahren nach Anspruch 1 oder 2, dadurch **gekennzeichnet,** daß in der allgemeinen Formel (I) $R^3$ für ein Wasserstoffatom steht.

4. Verfahren nach einem der Ansprüche 1 bis 3, dadurch **gekennzeichnet**, daß in der allgemeinen Formel (I) $R^4$ und $R^5$, die gleich oder verschieden sein können, jeweils für ein Wasserstoffatom oder eine $C_{1-3}$-Alkylgruppe stehen.

5. Verfahren nach einem der Ansprüche 1 bis 4, dadurch **gekennzeichnet**, daß in der allgemeinen Formel (I) $R^6$ für eine Gruppe $-CO_2R^7$ oder $-COR^7$ (worin $R^7$ wie in Anspruch 1 definiert ist) steht.

6. Verfahren nach einem der Ansprüche 1 bis 5, dadurch **gekennzeichnet**, daß in der allgemeinen Formel (I) $R^7$ für eine $C_{1-3}$-Alkylgruppe oder eine Phenylgruppe steht.

7. Verfahren nach Anspruch 1, dadurch **gekennzeichnet,** daß in der allgemeinen Formel (I) $R^1$ für eine Methylgruppe steht, $R^2$ und $R^3$ beide für ein Wasserstoffatom stehen, $R^4$ und $R^5$ beide für eine Methylgruppe stehen und $R^7$ für eine Methyl-, Ethyl- oder Phenylgruppe steht.

8. Verfahren nach Anspruch 1, dadurch **gekennzeichnet,** daß das Produkt aus Methyl-3-[2-(dimethylamino)ethyl]-5-[[(methylamino)-sulfonyl]methyl]-1H-indol-1-carboxylat ;
1-Acetyl-3-[2-(dimethylamino)ethyl]-N-methyl-1H-indol-5-methan-sulfonamid
und physiologisch annehmbaren Salzen und Solvaten davon ausgewählt wird.

9. Verfahren nach einem der Ansprüche 1 bis 8, dadurch **gekennzeichnet**, daß die Acylierungsstufe (A) in Gegenwart einer Base bei einer Temperatur von −10°C bis +150°C in Gegenwart eines Lösungsmittels oder in einem Zweiphasen-Reaktionsmedium in Gegenwart eines Phasentransferkatalysators durchgeführt wird.

10. Verfahren nach einem der Ansprüche 1 bis 8, dadurch **gekennzeichnet**, daß in Stufe (B) eine Verbindung der allgemeinen Formel (I), worin einer oder mehrere aus $R^1$, $R^2$, $R^4$ und $R^5$ für Alkylgruppen stehen, aus einer entsprechenden Verbindung der Formel (I), worin einer oder mehrere aus $R^1$, $R^2$, $R^4$ und $R^5$ für Wasserstoffatome stehen, durch Reaktion mit einem Alcylierungsmittel hergestellt wird.

## Revendications

### Revendications pour les Etats contractants BE CH DE FR GB IT LI NL SE :

1. Composé de formule générale (I) :

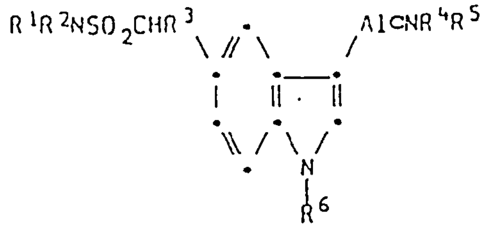

(I)

où $R^1$ représente un atome d'hydrogène ou un groupe alcoyle en $C_1$ à $C_6$ ou alcényle en $C_3$ à $C_6$ ; $R^2$ représente un atome d'hydrogène, un groupe alcoyle en $C_1$ à $C_3$ ou alcényle en $C_3$ à $C_6$, un groupe phényle ou phén(alcoylène en $C_1$ à $C_4$), ou un groupe cycloalcoyle en $C_5$ à $C_7$ ; $R^3$ représente un atome d'hydrogène ou un groupe alcoyle en $C_1$ à $C_3$ ; $R^4$ et $R^5$, qui peuvent être identiques ou différents, représentent chacun un atome d'hydrogène ou un groupe alcoyle en $C_1$ à $C_3$ ou 2-propényle, ou $R^4$ et $R^5$ ensemble forment un groupe benzylidène ; $R^6$ représente un groupe $-CO_2R^7$, $COR^7$, $-COCO_2R^7$, ou $-CONHR^7$, où $R^7$ représente un atome d'hydrogène, un groupe alcoyle en $C_1$ à $C_4$, un groupe cycloalcoyle en $C_3$ à $C_7$, un groupe alcényle en $C_2$ à $C_4$, ou un groupe phényle ou phén(alcoylène en $C_1$ à $C_4$) (sous les réserves que (a) $R^7$ ne représente pas un atome d'hydrogène ou un groupe benzyle lorsque $R^6$ est le groupe $-CO_2R^7$ et (b) $R^7$ ne représente pas un groupe alcényle lorsque $R^6$ est le groupe $-CONHR^7$) ; et Alc représente une chaîne alcoylène contenant deux ou trois atomes de carbone qui peuvent être non substitués ou substitués par au plus deux groupes alcoyle en $C_1$ à $C_3$, et ses sels et produits de solvatation physiologiquement acceptables.

2. Composé selon la revendication 1 dans lequel, dans la formule générale (I), $R^1$ représente un atome d'hydrogène ou un groupe alcoyle en $C_1$ à $C_6$ et $R^2$ représente un atome d'hydrogène ou un groupe alcoyle en $C_1$ à $C_3$, alcényle en $C_3$ à $C_6$ ou phén(alcoyle en $C_1$ à $C_4$).

3. Composé selon la revendication 1 ou 2, dans lequel, dans la formule générale (I), $R^3$ représente un atome d'hydrogène.

4. Composé selon l'une quelconque des revendications 1 à 3, dans lequel, dans la formule générale (I), $R^4$ et $R^5$, qui peuvent être identiques ou différents, représentent chacun un atome d'hydrogène ou un groupe alcoyle en $C_1$ à $C_3$.

5. Composé selon l'une quelconque des revendications 1 à 4, dans lequel, dans la formule générale (I), $R^6$ représente un groupe $-CO_2R^7$ ou $-COR^7$ (où $R^7$ est tel que défini dans la revendication 1).

6. Composé selon l'une quelconque des revendications 1 à 5, dans lequel, dans la formule générale (I), $R^7$ représente un groupe alcoyle en $C_1$ à $C_3$ ou un groupe phényle.

7. Composé selon la revendication 1 dans lequel, dans la formule générale (I), $R^1$ représente un groupe méthyle, $R^2$ et $R^3$ représentent tous deux un atome d'hydrogène, $R^4$ et $R^5$ représentent tous deux un groupe méthyle et $R^7$ représente un groupe méthyle, éthyle ou phényle.

8. Composé selon la revendication 1 choisi parmi : méthyl-3-[2-(diméthylamino)éthyl]-5[[(méthylamino)sulfonyl]méthyl]-1H-indole-1-carboxylate ;
1-acétyl-3-[2(diméthylamino)éthyl]-N-méthyl-1H-indole-5-méthane-sulfonamide,
et leurs sels et produits de solvatation physiologiquement acceptables.

9. Composition pharmaceutique pour l'administration orale, rectale ou intranasale comprenant au moins un composé de formule générale (I) tel que défini dans la revendication 1, ou un de ses sels ou produits de solvatation physiologiquement acceptables, avec un support ou excipient physiologiquement acceptable à cet effet.

10. Procédé de préparation d'un composé de formule générale (I) tel que défini dans la revendication 1 dans lequel :

(A) on acyle un composé de formule générale (II) :

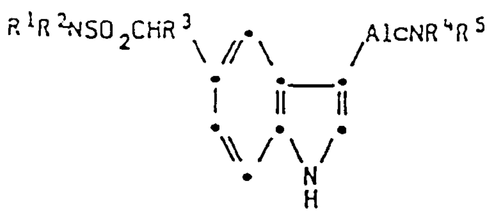

(II)

(où $R^1$, $R^2$, $R^3$, $R^4$ et $R^5$ sont tels que définis dans la revendication 1) ou un de ses dérivés protégés ; ou

(B) on soumet un composé de formule générale (I) ou un de ses sels ou dérivés protégés à une réaction d'interconversion pour former un autre composé de formule générale (I) ou un de ses sels ou dérivés protégés physiologiquement acceptables ; ou

(C) on soumet un dérivé protégé d'un composé de formule générale (I) ou un de ses sels à une réaction pour enlever le ou les groupe(s) protecteur(s) pour préparer un composé de formule générale (I) ou un de ses sels physiologiquement acceptables ; et, si nécessaire ou si on le désire, on soumet un composé préparé par l'étape (A) ou l'étape (B) à une ou deux autres réactions comprenant

(i) l'enlèvement des groupes protecteurs éventuellement présents ; et

(ii) on transforme un composé de formule générale (I) ou un de'ses sels en un sel ou produit de solvatation physiologiquement acceptable.

**Revendications pour les Etats contractants AT ES :**

1. Procédé de préparation d'un composé de formule générale (I) :

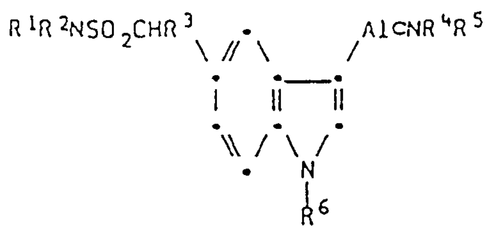

(I)

où $R^1$ représente un atome d'hydrogène ou un groupe alcoyle en $C_1$ , à $C_6$ ou alcényle en $C_3$, à $C_6$ ;
$R^2$ représente un atome d'hydrogène, un groupe alcoyle en $C_1$ à $C_3$ ou alcényle en $C_3$ à $C_6$, un groupe phényle ou phén(alcoylène en $C_1$ à $C_4$), ou un groupe cycloalcoyle en $C_5$ à $C_7$ ;
$R^3$ représente un atome d'hydrogène ou un groupe alcoyle en $C_1$ à $C_3$ ;
$R^4$ et $R^5$, qui peuvent être identiques ou différents, représentent chacun un atome d'hydrogène ou un groupe alcoyle en $C_1$ à $C_3$ ou 2-propényle, ou $R^4$ et $R^5$ ensemble forment un groupe benzylidène ;
$R^6$ représente un groupe $-CO_2R^7$, $COR^7$, $-COCO_2R^7$, ou $-CONHR^7$, où $R^7$ représente un atome d'hydrogène un groupe alcoyle en $C_1$ à $C_4$, un groupe cycloalcoyle en $C_3$ à $C_7$, un groupe alcényle en $C_2$ à $C_4$, ou un groupe phényle ou phén(alcoylène en $C_1$ à $C_4$) (sous les réserves que (a) $R^7$ ne représente pas un atome d'hydrogène ou un groupe benzyle lorsque $R^6$ est le groupe $-CO_2R^7$ et (b) $R^7$ ne représente pas un groupe alcényle lorsque $R^6$ est le groupe $-CONHR^7$) ; et Alc représente une chaîne alcoylène contenant deux ou trois atomes de carbone qui peuvent être non substitués ou substitués par au plus deux groupes alcoyle en $C_1$ à $C_3$, et ses sels et produits de solvatation physiologiquement acceptables, dans lequel :

(A) on acyle un composé de formule générale (II) :

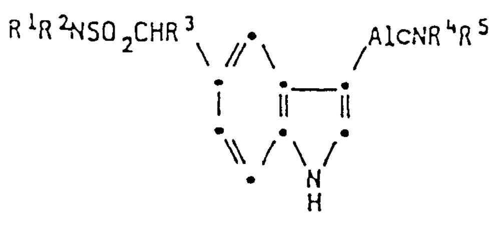

(II)

(où $R^1$, $R^2$, $R^3$, $R^4$ et $R^5$ sont tels que définis dans la revendication 1) ou un de ses dérivés protégés ; ou

(B) on soumet un composé de formule générale (I) ou un de ses sels ou dérivés protégés à une réaction d'interconversion pour former un autre composé de formule générale (I) ou un de ses sels ou dérivés protégés physiologiquement acceptables ; ou

(C) on soumet un dérivé protégé d'un composé de formule générale (I) ou un de ses sels à une réaction pour enlever le ou les groupe(s) protecteur(s) pour préparer un composé de formule générale (I) ou un de ses sels physiologiquement acceptables ; et, si nécessaire ou si on le désire, on soumet un composé préparé par l'étape (A) ou l'étape (B) à une ou deux autres réactions comprenant

(i) l'enlèvement des groupes protecteurs éventuellement présents ; et

(ii) on transforme un composé de formule générale (I) ou un de ses sels en un sel ou produit de solvatation physiologiquement acceptable.

3. Procédé selon la revendication 1 ou 2, dans lequel, dans la formule générale (I), $R^3$ représente un atome d'hydrogène.

4. Procédé selon l'une quelconque des revendications 1 à 3, dans lequel, dans la formule générale (I), $R^4$ et $R^5$, gui peuvent être identiques ou différents, représentent chacun un atome d'hydrogène ou un groupe alcoyle en $C_1$ à $C_3$.

5. Procédé selon l'une quelconque des revendications 1 à 4, dans lequel, dans la formule générale (I), $R^6$ représente un groupe $-CO_2 R^7$ ou $-COR^7$ (où $R^7$ est tel que défini dans la revendication 1).

6. Procédé selon l'une quelconque des revendications 1 à 5, dans lequel, dans la formule générale (I), $R^7$ représente un groupe alcoyle en $C_1$ à $C_3$ ou un groupe phényle.

7. Procédé selon la revendication 1 dans lequel, dans la formule générale (I), $R^1$ représente un groupe méthyle, $R^2$ et $R^3$ représentent tous deux un atome d'hydrogène, $R^4$ et $R^5$ représentent tous deux un groupe méthyle et $R^7$ représente un groupe méthyle, éthyle ou phényle.

8. Procédé selon la revendication 1, dans lequel le composé est choisi parmi :
méthyl-3-[2-(diméthylamino)éthyl]-5[[(méthylamino)sulfonyl]méthyl]-1H-indole-1-carboxylate ;
1-acétyl-3-[2(diméthylamino)éthyl]-N-méthyl-1H-indole-5-méthane-sulfonamide,
et leurs sels et produits de solvatation physiologiquement acceptables.

9. Procédé selon l'une quelcongue des revendications 1 à 8, dans lequel on conduit l'étape d'acylation (A) en présence d'une base à une température allant de −10 à +150°C en présence d'un solvant ou dans un milieu réactionnel à deux phases en présence d'un catalyseur de transfert de phase.

10. Procédé selon l'une des revendications 1 à 8, dans lequel dans l'étape B on prépare un composé de formule générale (I), dans laquelle un ou plusieurs des groupes $R^1$, $R^2$, $R^4$ et $R^5$ sont des groupes alcoyle, à partir d'un composé correspondant de formule (I) dans laquelle un ou plusieurs des groupes $R^1$, $R^2$, $R^4$ et $R^5$ représentent des atomes d'hydrogène, par réaction avec un agent d'alcoylation.